# EUROPEAN PATENT APPLICATION

(11) **EP 2 312 316 A1**
(43) Date of publication of application: **20.04.2011**
(21) Application number: 10178275.3
(22) Date of filing: 17.08.2000
(51) Int. Cl.: G01N 33/574

(54) **Anti-EPHA2 antibodies as a cancer diagnostic**

(30) Priority: 17.08.1999 US 149259 P
(62) Divisional of application: 00955686.1
(71) Applicant: Purdue Research Foundation, West Lafayette, IN 47906 (US); GLAXO GROUP LIMITED, Greenford, Middlesex UB6 0NN (GB); THE UNIVERSITY OF NORTH CAROLINA AT CHAPEL HILL, Chapel Hill, NC 27599-4105 (US)
(72) Inventor: Kinch, Michael S., Laytonsville, MD 20882 (US); Zantek, Nicole Dodge, Edina, MN 55435 (US)
(74) Representative: Vossius & Partner

(57) **Abstract**

Method and kits are provided for the detection and diagnosis of metastatic disease. More particularly, the methods and kits employ compounds that can detect EphA2, a specific epithelial cell tyrosine kinase that is overexpressed in metastatic tumor cells. In one embodiment the compound is an antibody capable of binding to an epitope of EphA2.

## Description

### Field of The Invention

The present invention relates to diagnosis of metastatic disease. More particularly, this invention relates to reagents that can detect a specific epithelial cell tyrosine kinase that is overexpressed in metastatic tumor cells. Most particularly, this invention relates to reagents which bond to an intracellular epitope of the epithelial cell tyrosine kinase, and the use of these reagents for cancer diagnosis.

### Background And Summary of The Invention

Cancer cell metastasis requires cellular capacity to 1) detach from a primary tumor, 2) migrate and invade through local tissues, 3) translocate to distant sites in the body (via lymph or blood), 4) colonize a foreign site, and 5) grow and survive in this foreign environment. All of these behaviors are linked to cell adhesions. Cell adhesions control the physical interactions of cells with their microenvironment. Cell adhesions also initiate signals that dictate tumor cell growth, death, and differentiation.

Various cancer cells, including breast cancer cells, are known to exhibit altered cell adhesion. As compared to normal breast epithelia, transformed human breast epithelial cells have decreased cell-cell contacts and increased interactions with the surrounding extracellular matrix. These changes facilitate increased detachment and migration of cancer cells away from cell colonies and are directly linked with alteration in tyrosine phosphorylation of cell membrane proteins. Tyrosine phosphorylation is a potent form of cell signal transduction, and alteration in levels of tyrosine phosphorylation is believed to be important for tumor cell invasiveness. Thus, regulation of tyrosine phosphorylation represents a promising target for therapeutic intervention against metastatic cancer. Tyrosine phosphorylation is controlled by cell membrane tyrosine kinases, and increased expression of tyrosine kinases is known to occur in metastatic cancer cells.

Identification of increased expression of cell membrane tyrosine kinases would aid in the diagnosis and treatment of metastatic diseases. One such tyrosine kinase is EphA2. A member of the Eph family of tyrosine kinases known as Ephrins, EphA2 is a transmembrane receptor tyrosine kinase with a cell-bound ligand. Although cloned a decade ago, see Lindberg, R.A. and Hunter, T., "cDNA Cloning and Characterization of Eck, an Epithelial Cell Receptor Protein-tyrosine Kinase in the Eph/elk Family of Protein Kinases," Mol. Cell. Biol. 10 (12), 6316-6324 (1990), rather little is known about EphA2 function, largely because EphA2-specific antibodies previously have been difficult to generate.

To facilitate research on EphA2, an improved method for generating a panel of monoclonal antibodies specific for tyrosine phosphorylated proteins has been developed. Using this method, a multiplicity of EphA2 recognizing monoclonal antibodies has been generated. These antibodies have been used to show that EphA2 is overexpressed in metastatic breast, lung, colon, and prostate cells. Because EphA2 is expressed differently in normal and metastatic cells, EphA2-specific antibodies are useful in the diagnosis of metastatic disease. Antibodies produced by one particular hybridoma recognize an intracellular epitope of EphA2 and have been shown to be highly specific in binding to EphA2.

Thus, one aspect of this invention is a compound which specifically binds to an intracellular epitope of EphA2. In a preferred embodiment, the compound is an antibody specific for a domain of the EphA2 protein. However, natural or artificial ligands, peptides, anti-sense, ATP analogies, or other small molecules capable of specifically targeting EphA2 may be employed. A second aspect of this invention is a method for generating antibodies which recognize EphA2 intracellular epitopes. Another aspect of this invention is the use of EphA2-specific antibodies in the diagnosis of metastatic disease. An additional aspect of this invention is a diagnostic reagent specific for detecting EphA2, any fragment thereof, or DNA or RNA coding for the EphA2 protein. A further aspect of this invention is a kit comprising an antibody capable of specific binding to an epitope of EphA2 and means for detecting antibody-EphA2 binding.

Additional features of the present invention will become apparent to those skilled in the art upon consideration of the following detailed description of preferred embodiments exemplifying the best mode of carrying out the invention as presently perceived.

### Brief Description of The Drawings

Fig. 1A-C show a series of western blots showing EphA2 expression in cell lines derived from human prostate cells;
Fig. 1A is a western blot showing EphA2 expression in various human prostate cancer cell lines;
Fig. 1B is a western blot showing EphA2 expression in human prostatic epithelial cell line MLC and expression in that cell line after transformation by oncogenic K-Ras or X-irradiation;
Fig. 1C is similar to Fig. 1B, except showing expression in human prostatic epithelial cell line 267B1 and expression in that cell line after transformation by oncogenic K-Ras or X-irradiation;
Fig. 2 is a western blot showing EphA2 expression in various human mammary epithelial cell lines;
Fig. 3A-B shows EphA2 localization in the cell membranes of various mammary epithelial cell lines, as seen by immunofluorescence microscopy;

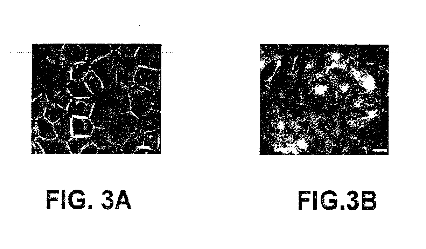

Fig. 3A shows EphA2 localization in sites of cell adhesion in normal MCF-10A cells; and
Fig. 3B shows EphA2 redistribution in malignant cells.

### Detailed Description of the Invention

Antibodies specific for EphA2 have been isolated through an improved method. The method employed is designed for increased sensitivity and diversity of responding hybridomas. According to this method, tyrosine phosphorylated proteins from Ras-transformed human epithelial cells are isolated by affinity chromatography using existing phosphotyrosine-specific antibodies. The tyrosine phosphorylated proteins are then used as an immunogen for producing monoclonal antibodies. Low-dose amounts of tyrosine phosphorylated proteins are injected proximal to lymph nodes, every other day, over a ten day period (the RIMMS strategy). B cells from engorged lymph nodes are then isolated and fused with a Bcl-2-overexpressing myeloma, to minimize apoptosis after fusion. This method results in increased diversity, specificity, and cost-effectiveness of hybridoma production. The hybridomas are first screened to identify those hybridomas producing antibodies capable of distinguishing malignant from normal cancer cells. To date, at least 450 such hybridomas have been identified.

Hybridomas which are specific to EphA2 have been selected. Use of the RIMMS strategy has resulted in the production of various monoclonal antibodies that specifically bind EphA2. Of the first four hybridomas characterized, two recognize independent epitopes on EphA2. The first, D7, recognizes an intracellular epitope. The second, B2D6, binds to an extracellular epitope. D7 has proven to be highly specific for an intracellular epitope of EphA2 and this specificity provides much of the current basis for diagnosis of metastatic tumors.

It is known in the art to use antibodies to detect the presence or overexpression of a specific protein. Because EphA2 is overexpressed in metastatic cells, EphA2-specific antibodies of this invention may be used to detect this overexpression and, thus, to detect metastatic disease. Such techniques include but are not limited to western blotting, dot blotting, precipitation, agglutination, ELISA assays, immunohistochemistry, in situ hybridization, flow cytometry on a variety of tissues or bodily fluids, and a variety of sandwich assays. These techniques are well known in the art. See, for example, U.S. Patent No. 5,876,949, hereby incorporated by reference. When antibodies specific for an intracellular epitope of EphA2 are used, the cells must be lysed and incubated with the antibody. The above techniques may be performed on whole-cell lysates, or EphA2 may be separated out for testing, such as by immunoprecipitation. The D7 antibodies of this invention are highly specific for an intracellular epitope of EphA2 and have proven to be sensitive to differential expression of EphA2 in metastatic cells. Other techniques, such as immunohistological staining, require whole cells, and may further require cell layers of a particular cell density. Such tests require an antibody specific for an extracellular epitope of EphA2

The antibodies of this invention may be used to detect metastatic disease in a wide variety of tissue samples. For instance, research using EphA2-specific antibodies has revealed that altered EphA2 expression occurs in breast, kidney, prostate, lung, and colon cells, and it is believed that altered EphA2 expression occurs in other types of cell metastasis, particularly epithelial malignancies. EphA2-specific antibodies may be used to detect metastasis in biopsied tumor tissue. Also, samples of a variety of body fluid samples, such as blood, plasma, spinal fluid, saliva, and urine, can be tested with the antibodies of the present invention. Altered EphA2 expression in these samples indicates the presence of metastatic disease.

Additionally, other antibodies may be used in combination with the antibodies of the present invention to provide further information concerning metastatic disease state. For example, the EphA2 of metastatic cells exhibits altered tyrosine phosphorylation. In normal breast epithelial cells, EphA2 is expressed and is tyrosine phosphorylated. However, in metastatic breast epithelial cells, EphA2 is overexpressed, and the EphA2 is not tyrosine phosphorylated. Because a test quantifying EphA2 expression sometimes may lead to an ambiguous result, it may be desirable to determine tyrosine phosphorylation, as well as the magnitude of EphA2 expression. Thus, a method of diagnosis using the antibodies of this invention in combination with phosphotyrosine-specific antibodies provides data for determining the state of metastatic disease.

Moreover, the EphA2-specific antibodies of this invention can be exploited to detect changes in EphA2 localization which are associated with metastasis. In normal breast and prostate epithelial cells, EphA2 is enriched in within cites of cell adhesion. Conversely, in metastatic prostate cells EphA2 is diffusely distributed, and in metastatic breast cancer cells EphA2 is redistributed into the membrane ruffles. Techniques such as immunohistological staining or immunofluorescent microscopy are well known in the art and may be used to visualize EphA2 distribution. See, for example, U.S. Patent No. 5,514,554, hereby incorporated by reference. EphA2 expression can be detected by using antibodies capable of detecting whole EphA2 or fragments of the EphA2 protein. Other methods of detecting altered EphA2 expression include detecting DNA or RNA sequences coding for the EphA2 protein.

In order to detect overexpression or altered distribution of EphA2, the EphA2-specific antibodies may be labeled covalently or non-covalently with any of a number of known detectable labels, such fluorescent, radioactive, or enzymatic substances, as is known in the art. Alternatively, a secondary antibody specific for the antibodies of this invention is labeled with a known detectable label and used to detect the EphA2-specific antibodies in the above techniques.

Preferred labels include chromogens dyes. Among the most commonly used are 3-amino-9-ethylcarbazole (AEC) and 3,3'-diaminobenzidine tetrahydrocholoride (DAB). These can be detected using light microscopy. Also preferred are fluorescent labels. Among the most commonly used fluorescent labeling compounds are fluorescein isothiocyanate, rhodamine, phycoerythrin, phycocyanin, allophycocyanin, o-phthaldehyde and fluorescamine. Chemiluminescent and bioluminescent compounds such as luminol, isoluminol, theromatic acridinium ester, imidazole, acridinium salt, oxalate ester, luciferin, luciferase, and aequorin also may be used. When the fluorescent-labeled antibody is exposed to light of the proper wavelength, its presence can be detected due to its fluorescence.

Also preferred are radioactive labels. Radioactive isotopes which are particularly useful for labeling the antibodies of the present invention include ³H, ¹²⁵I, ¹³¹I, ³⁵S, ³²P, and ¹⁴C. The radioactive isotope can be detected by such means as the use of a gamma counter, a scintillation counter, or by autoradiography.

Another method in which the antibodies can be detectably labeled is by linking the antibodies to an enzyme and subsequently using the antibodies in an enzyme immunoassay (EIA) or enzyme-linked immunosorbent assay (ELISA). The enzyme, when subsequently exposed to its substrate, reacts with the substrate and generates a chemical moiety which can be detected, for example, by spectrophotometric, fluorometric, or visual means. Enzymes which can be used to detectably label antibodies include, but are not limited to malate dehydrogenase, staphylococcal nuclease, delta-5-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase, and acetylcholinesterase. Other methods of labeling and detecting antibodies are known in the art and are within the scope of this invention.

### Example 1

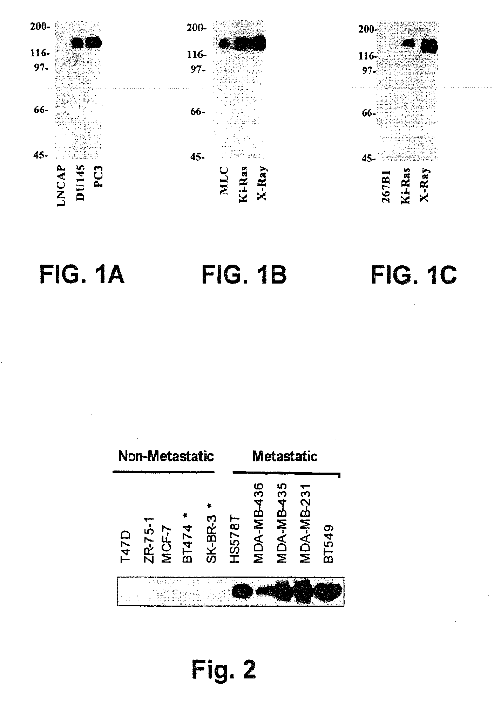

The antibodies produced by the D7 hybridoma are used to detect differential expression of EphA2 between normal prostate epithelial cells and metastatic cells. Fig. 1 shows EphA2 expression in various human prostate cell lines. Referring first to Fig. 1A, three metastatic cell lines, LNCAP, DU145, and PC3, are tested for levels of EphA2 expression. It is known that, of these three cell lines, LNCAP is the least invasive, DU145 is somewhat more invasive, and PC3 is the most invasive. EphA2 expression is determined by western blotting with D7 antibodies. As can be seen in Fig. 1A, EphA2 expression positively correlates with invasiveness.

In Fig. 1B, D7 antibodies are used to test EphA2 expression in normal MLC cells as compared to expression in transformed cells. Normal MLC cells, MLC cells which have been transformed by K-Ras, and MLC cells with have been transformed by X-irradiation are studied. As can be seen in Fig. 1B, EphA2 is overexpressed in both of the transformed cell lines. Fig. 1C shows results similar to Fig. 1B, except the normal cell line is 267B1. As with Fig. 1B, Fig. 1C shows that EphA2 is overexpressed in the transformed cells. In sum, Fig. 1 demonstrates that EphA2-specific antibodies detect changes in metastatic cells, and that tests using these antibodies indicate the level of metastatic invasiveness.

### Example 2

EphA2 antibodies are used to detect altered EphA2 expression in metastatic mammary cells. EphA2 is expressed in normal mammary epithelial cells. Fig. 2 illustrates altered EphA2 expression in mammary tumor cell lines. As can be seen in Fig. 2, western blots from whole cell lysates using D7 antibodies reveal that EphA2 expression is completely absent in cells derived from non-metastatic breast tumors (ZR75-1, BT474, SKBR3, MDA-MB-435). By contrast, EphA2 is overexpressed in metastatic breast cancer cell lines (MDA-MB-435, MDA-MB-231). Thus, EphA2 antibodies detect altered EphA2 expression in breast cancer cells, which can be used to diagnose metastasis. Moreover, in non-metastatic breast epithelial cells, loss of EphA2 occurs early in the disease, and testing with EphA2-specific antibodies provide information relevant to invasiveness even when other known markers remain normal. Thus, D7 antibodies are useful as a diagnostic, even in early stages of disease.

### Example 3

EphA2 antibodies in combination with other antibodies are used to detect further alterations in EphA2 expression. As discussed above in Example 2, western blots using D7 can distinguish between non-metastatic and metastatic tumors, with non-metastatic tumors failing to express EphA2, and metastatic cells overexpressing EphA2. However, different results are found when tyrosine phosphorylation is studied. Using a phosphotyrosine-specific antibody, it has been found that EphA2 is phosphorylated in normal cells, but it is not phosphorylated in metastatic cells. Thus, while EphA2 specific antibodies can qualitatively detect a difference between metastatic and non-metastatic mammary tumor cells, diagnostics incorporating both an EphA2-specific antibody and a phosphotyrosine-specific antibody provides a sensitive test for distinguishing between normal, non-metastatic, and metastatic mammary cells.

### Example 4

Immunofluorescently labeled EphA2-specific antibodies detect redistribution of EphA2 expression in transformed cells. The EphA2-specific antibodies used in this example are produced by a cell line known as B2D6, and these antibodies are specific for an extracellular epitope of EphA2. As seen in Fig. 3A, immunofluorescence with B2D6 demonstrates that EphA2 is found within sites of cell-cell contact in normal cells. However, in transformed cells, shown in Fig. 3B, EphA2 is redistributed. Furthermore, in metastatic cells EphA2 is found in the membrane ruffles. Similarly, in normal prostate epithelial cells, EphA2 is found within sites of cell-cell adhesion, but in metastatic prostate epithelial cells, EphA2 is overexpressed and the expression is diffusely distributed. Therefore, immunofluorescence using EphA2-specific antibodies provides an additional means for diagnosing the transformation and metastatic state of tumor cells.

As shown in Examples 1-4, overexpression, redistribution, and phosphorylation of EphA2 in metastatic cells provide various bases for diagnosis of metastatic tumors using EphA2-specific antibodies. Immunohistochemistry or Western blotting may be used to monitor the change of EphA2 expression in biopsied samples of patient breast tissue, prostate tissue, or tissue from other tumors. Additionally, D7 and other EphA2-specific antibodies can be used to monitor plasma, urine, and other body fluids to detect altered expression of EphA2, which would signal metastasis. Detection of altered tyrosine phosphorylation of EphA2 in combination with information concerning an alteration of EphA2 expression further aids in diagnosis of metastatic disease.

Although the invention has been described in detail with reference to preferred embodiments, variations and modifications exist within the scope and spirit of the invention as described and defined in the following claims.

Furthermore, the present invention relates to the following items:
1. A method for detecting the presence of metastatic cells in a cell population comprising the steps of
   lysing at least a portion of the cell population,
   incubating the lysed cells with a reagent capable of specific binding to an epitope of EphA2 to allow antibody binding to said epitope, and
   detecting compound-epitope binding.
2. The method of item 1 wherein the reagent is an antibody.
3. The method of item 2 wherein the epitope of EphA2 is an intracellular epitope of EphA2.
4. The method of item 3 wherein the antibody is produced by hybridoma cell line D7.
5. The method of item 2 wherein the antibody is labeled with a detectable label, and the detecting step includes detecting the label.
6. The method of item 5 wherein the antibody is labeled with a fluorescent label and the detecting step comprises detecting the fluorescent label.
7. The method of item 5 wherein the antibody is labeled with a radioactive label and the detecting step comprises detecting the radioactive label.
8. The method of item 1 wherein the cell population comprises cells from a breast or prostate tissue biopsy.
9. The method of item 1 wherein the cell population is harvested from a body fluid selected from the group consisting of blood, plasma, spinal fluid, saliva, and urine.
10. The method of item 9 wherein the detecting step includes a diagnostic method selected from the group consisting of ELISA assays and flow cytometry.
11. The method of item 1 wherein the incubating and detecting steps comprise western blotting methodology.
12. The method of item 11 further comprising the steps of
   providing a second antibody having phosphotyrosine specificity, and western blotting with the second antibody.
13. The method of item 1 wherein the metastatic cells are selected from the group consisting of breast, prostate, lung, and colon cancers.
14. A method of producing an antibody which specifically binds to an intracellular epitope of EphA2 comprising the steps of
   injecting tyrosine phosphorylated proteins into lymph nodes of a mammal,
   harvesting lymph node cells from the mammal,
   fusing lymph node cells with myeloma cells to form hybridomas, selecting at least one hybridoma producing an antibody which binds to the intracellular epitope of EphA2,
   isolating the antibody.
15. The method of item 14 wherein the antibody recognizes an antigen also recognized by the monoclonal antibody D7.
16. The method of item 14 wherein the tyrosine phosphorylated proteins are EphA2.
17. The antibody produced by the method of item 14.
18. An antibody which specifically binds to an intracellular epitope of EphA2.
19. The antibody of item 18 bound to a detectable labeled.
20. The antibody of item 19 which is produced by hybridoma cell line D7.
21. A method for detecting the presence of metastatic cells in a cell population comprising the steps of
   incubating the cells with a reagent capable of specific binding to a compound associated with EphA2 expression, and
   detecting reagent-compound binding.
22. The method of item 21 wherein the reagent is an antibody.
23. The method of item 21 wherein the compound is selected from the group consisting of EphA2, a fragment of EphA2, DNA coding for the EphA2 protein, and RNA coding for the EphA2 protein.
24. The method of item 21 further comprising the step of fixing the cells on a slide, and the detecting step comprises immunofluorescence staining.
25. A kit for detecting the presence of metastatic cells in a cell population comprising
   an antibody capable of specific binding to an epitope of EphA2, and means for detecting antibody-epitope binding.
26. The kit of item 25 wherein the means for detecting antibody-epitope binding is a label bound to the antibody.
27. The kit of item 25 further comprising an antibody having phosphotyrosine specificity.

## Claims

1. A method of detecting the presence of metastatic cancer cells in a selected cell population comprising:
assaying at least a portion of the selected cell population for EphA2 overexpression, wherein overexpression of EphA2 is indicative of the presence of a metastatic cancer cell in the selected cell population.

2. The method of claim 1 further comprising assaying the portion of the selected cell population for EphA2 tyrosine phosphorylation, wherein lack of tyrosine phosphorylation of EphA2 is indicative of the presence of a metastatic cancer cell in the selected cell population.

3. A method of detecting the presence of metastatic cancer cells in a selected cell population comprising:
assaying at least a portion of the selected cell population for the distribution of EphA2 in a cell, wherein changes in EphA2 localization from within sites of cell adhesion are indicative of metastasis.

4. The method of any one of claims 1 to 3 comprising:
(a) incubating the cells with
(i) a reagent capable of specific binding to EphA2; or
(ii) a reagent capable of specific binding to an DNA encoding at least a portion of an EphA2 protein
to allow binding of the reagent to EphA2 or the RNA; and
(b) detecting reagent binding to EphA2 or the RNA; and
(c) detecting expression of EphA2.

5. The method of claim 4, wherein the reagent capable of specific binding is an antibody.

6. The method of claim 4 or 5 comprising lysing at least a portion of the cell population prior to incubation with the reagent.

7. The method of claim 4 or 5 comprising assaying whole cells.

8. The method of claim 6, wherein the antibody is capable of specific binding to an intracellular epitope of EphA2.

9. The method of any one of claims 4, 5, 7 and 8, wherein the antibody is capable of specific binding to an extracellular epitope of EphA2.

10. The method of any one of claims 1 to 9, wherein the cell population comprises cells selected from the group consisting of breast cells, kidney cells, prostate cells, lung cells and colon cells.

11. The method of any one of claims 1 to 10, wherein the cell population comprises epithelial cells.

12. The method of claim 10 or 11 wherein the cells are cancer cells.

13. The method of any one of claims 1 to 12, wherein the cell population comprises cells from a tissue biopsy.

14. A kit for detecting the presence of metastatic cells in a cell population comprising:
(a) an EphA2-specific antibody, and
(b) means for detecting antibody-epitope binding.

15. The kit of claim 14 wherein the means for detecting antibody-epitope binding is a label bound to the antibody.
